(19) Europäisches Patentamt
European Patent Office
Office européen des brevets

(11) Publication number: **0 386 237 B1**

# (12) EUROPEAN PATENT SPECIFICATION

(45) Date of publication of patent specification: **14.12.94**

(51) Int. Cl.5: **C12Q 1/32**, C12Q 1/28

(21) Application number: **88907812.7**

(22) Date of filing: **02.09.88**

(86) International application number:
**PCT/JP88/00881**

(87) International publication number:
**WO 90/02817 (22.03.90 90/07)**

The file contains technical information submitted after the application was filed and not included in this specification

## (54) ENZYME ASSAY OF BIOLOGICAL SUBSTANCES.

(43) Date of publication of application:
**12.09.90 Bulletin 90/37**

(45) Publication of the grant of the patent:
**14.12.94 Bulletin 94/50**

(84) Designated Contracting States:
**DE FR GB IT**

(56) References cited:
**EP-A- 0 124 909**
**EP-A- 0 199 363**
**EP-A- 0 207 493**

**NIHON NOGEI KAGAKUKAI-HEN (Nihon Nogei Kagakukai ABC Series (4) Koso-Bio-technology eno Shishin-I), 20 March 1985, Asakura Shoten, Tokyo (JP); pp. 94-114/**

**TANPAKUSHITSU KAKUSAN KOSO RINJI ZOKAN-GO (Glutathione Kenkyu no Epoch), vol. 33, no. 9, July 1988, Tokyo (JP), Takahashi Kazuhiko (Selenium to Glutathioneperoxidase); pp. 1495-1504/**

(73) Proprietor: **ORIENTAL YEAST CO., LTD.**
**6-10, Azusawa 3-chome**
**Itabashi-ku**
**Tokyo 174 (JP)**

(72) Inventor: **FUJITA, Tuyosi**
**103, 2-16, Satsukigaoka 2-chome**
**Ikeda-shi Osaka-fu 563 (JP)**
Inventor: **TAKAGAHARA, Isamu**
**3-59, Fushimidai 5-chome**
**Inagawa-cho**
**Kawabe-gun Hyogo-ken 666-02 (JP)**

(74) Representative: **Thomsen, Dieter, Dr.**
**Patentanwalt**
**Postfach 70 19 29**
**D-81319 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The invention relates to an enzyme assay of biochemical substances and particularly to an enzyme assay for the content of hydrogen peroxide ( $H_2O_2$ ),i.e. a method of analyzing substances contained in body fluid ( e.g. blood and urine ) or biochemical substances generating $H_2O_2$ as a result of biochemical reaction.

In the conventional substrate or enzyme analysis using $H_2O_2$ in the detection system, a known technique to measure the amount of $H_2O_2$ is to allow a hydrogen donor to react with an electron or radical receptor ( coupler ) in the presence of a peroxidase ( oxydative condensation ) to form a colored substance by the action of $H_2O_2$, followed by colorimetry. As examples of the hydrogen donor, there may be mentioned dimethylaniline, diethylaniline, o-tolidine, o-toluidine, p-toluidine, o-phenylenediamine, N-ethyl-N-(3-methylphenyl-N'-succinylethylenediamine), N,N'-dimethyl-p-phenylenediamine, benzidine, o-anisidine, p-anisidine, dianisidine, o-cresol, p-chlorophenol, N,N-diethyl-m-toluidine, N-ethyl-N-(2-hydroxy-3-sulfopropyl)-m-toluidine ( TOOS ), m-cresol, $\alpha$-naphthol, $\beta$-naphthol, catechol, guaiacol, pyrogallol, 2,7-diaminofluorene, N-ethyl-N-(3-methylphenyl)-N'-acetylethylenediamine, leucoindophenol, 2,4-dichlorophenol and 2-hydroxy-3,5-dichlorobenzenesulfonic acid. Illustrative examples of the coupler include 2-thiophenecarboxylic acid hydrazine, benzidine, 3-methyl-2-benzothiazolinonehydrazone and 4-aminoantipyrine.

As the peroxide activator, may be used, besides peroxidase, tungstic acid, a salt thereof, molybdic acid, a salt thereof, a mixture of one of these inorganic compounds with iodide.

These methods, however, have the disadvantages that the sensitivity is low, the reagents used evolve disagreeable odor and are low in stability, the color to be measured is liable to be affected by hemoglobin, bilirubin and other pigment contained in test samples, and consistent data cannot be obtained when the blanks for reagent fluctuate because this method is based on relative measurment.

Another type of method is also known, in which $H_2O_2$ is allowed to react with ethanol or methanol by the action of a catalase, and the amount of acetaldehyde or formaldehyde formed is determined by adding acetaldehyde dehydrogenase or formaldehyde dehydrogenase and measuring the increased amount of NAD(P)H.

But correct analysis of acetaldehyde is difficult with test samples from drinkers who take much alcoholic beverages because of a lot of acetaldehyde is contained in the blood and urine. To avoid such effects of endogenous substances, the following techniques have been proposed and put into practice.

In the first technique, a catalase is used in the elimination step; it is inactivated by addition of sodium azide at the and of elimination, and an enzyme or substrate that generates $H_2O_2$ after reaction is than added, followed by colorimetry by the use of a peroxidase ( POD ) color-development system. These problems involved in this technique are that whether or not sodium azide is able to completely inactivate the catalase is uncertain and that oxidase in general ( including POD ) suffers inhibitory action.

In the second technique, an oxidase is allowed to act directly or indirectly in the presence of other type of enzyme or substrate, upon the endogenous substance in question; $H_2O_2$ thus formed is condensed with a hydrogen donor in the presence of POD to produce an inactive compound having no absorption in the visible region, thereby eliminating the endogenous substance, and thereafter measurement is started (JP-A-29159/1982).

Actually, however, the condensation product generally shows slight absorption in the visible region, causing positive errors particularly when measuring a small amount of body-fluid substances.

In the third technique according to JP-A-61-173799 (1986), a catalase is used in an amount less than ten times that of POD, thus eliminating the need for the use of sodium azide. In this case, however, measurement is started without inactivating the catalase used, and hence decomposition of $H_2O_2$ by the catalase during measurement is unavoidable, thus causing negative errors.

Thus, any of the techniques described above involves some problems.

Further methods for detection of biochemical substances are described in EP-A-207493, which discloses a method of terminating an isocitrate dehydrogenase reaction in a system wherein $NADP^+$ formed from NADPH is reproduced into NADPH in the conjoint presence of an isocitrate, metallic ions such as magnesium or manganese ions, and isocitrate dehydrogenase in the absence of ATP, AMP or ADP in an assay for assaying a substance by means of a reaction of NADPH to $NADP^+$, which comprises terminating the isocitrate dehydrogenase reaction by adding a chelating agent to the reaction system; and in EP-A-199363, which discloses a method of terminating an isocitrate dehydrogenase reaction in a system wherein $NAD^+$ formed from NADH is reproduced into NADH in the conjoint presence of an isocitrate, metallic ions such as magnesium or manganese ions, and isocitrate dehydrogenase in an assay for assaying a substance by means of a reaction of NADH to $NAD^+$, which comprises terminating the isocitrate dehydrogenase reaction by adding ATP and/or a chelating agent to the reaction system.

The 1. method is the elimination of endogenous substances that interfere the result by use of enzymes for converting said endogenous substances involving conversion of NAD(P)H to NAD(P) and regeneration of NAD(P)H by use of isocitric acid, metal ions and isocitrate dehydrogenase (iCDH);

and the 2. method is the conversion of the particular biochemical substance along the same route as in 1) by use of at least one additional enzyme (preferably the first on the route), the iCDH being inactivated by a chelating agent and/or ATP, making the conversion NAD(P)H to NAD(P) detectable by spectrophotometry at 340nm.

However, not all biochemically existing problems can be solved by the methods as described. Rather the biochemical practise offers a lot of different open points where individual solution ways are demanded. And furthermore, as stated above, the peroxidase method has the disadvantages of low sensitivity and incorrect measurement, while the catalase method suffers from the adverse effects of endogenous substances; and the techniques so far proposed to eliminate such endogenous substances give no satisfactory result.

Thus the object of the present invention is to improve the practical selection for particular methods in cases wherein the route of conversion includes the formation of hydrogen peroxide.

According to the invention this object is solved by the method of claim 1, i.e. an enzyme assay for the quantitative analysis of a biochemical substance which, when subjected to a predetermined enzyme or substrate, undergoes a reaction which, either directly or indirectly after adding one or more secondary enzymes or substrates, yields hydrogen peroxide as a reaction product, comprising:

adding to a test sample glutathione (GSH), glutathione peroxidase (GSHPOD), glutathione reductase (GR), NADPH or NADH, isocitric acid, metal ions, isocitrate dehydrogenase, and said one or more secondary enzymes or substrates, thereby consuming any endogenous substances which may generate hydrogen peroxide upon reaction with any of said added substances, or that would otherwise eventuate in the reduction of NADPH or NADH to $NADP^+$ or $NAD^+$, and regenerating NADPH or NADH at the same time;

inactivating the isocitrate dehydrogenase;

adding, simultaneously with or after said inactivating step, said predetermined enzyme or substrate that generates hydrogen peroxide as reaction product when reacting with the biological substance directly or indirectly after further reaction with said one or more secondary substances or enzymes, whereby any formed hydrogen peroxide will be subjected to the action of the GSHPOD in the presence of the GSH to produce oxidized glutathione (GSSG) and any formed GSSG will be subjected to the action of the GR to produce $NADP^+$ or $NAD^+$ from the NADPH or NADH previously added or regenerated; and

measuring at 340 nm any decrease of the NADPH or NADH.

A particular embodiment can be seen from claim 2, wherein said inactivating step comprises adding a sufficient amount of a chelating agent.

The invention demonstrates a novel method for the quantitative analysis of biochemical substances which uses $H_2O_2$ as reaction product and is free from the problems mentioned above. Specifically the invention shows a new enzyme assay under the notion that absorbance measurement in the ultraviolet region is the best in order to meet the general demand for an analytical method which can be simply performed without diluting test samples, is not adversely affected by any other substances, and can be applied to automatic analyzers.

The amounts of a variety of precursors participating in the formation of $H_2O_2$ contained in a test sample can be determined by the present invention. The invention includes the quantitative analysis of such precursors and related enzymes by treating the precursors with an oxidase and measuring the amount of $H_2O_2$ thus formed.

The practical realization of the invention is a method of measuring the amount of an enzyme or a substrate using $H_2O_2$ in the detection system, which comprises adding all the required enzymes or enzyme groups, or all the required substrates or substrate groups, to the detection system, allowing oxidase to act upon the reaction products to form $H_2O_2$, oxidizing reduced-form glutathione ( GSH ) into its oxidized form ( GSSG ) by the action of GSHPOD in the presence of $H_2O_2$, converting NADPH or NADH into $NAD^+$ or $NADP^+$ by the action of GSH reductase ( GR ), and measuring the change in absorbance in the ultraviolet region, thereby determining the amount of substrate or enzyme, wherein the endogenous substances contained in the test sample that participate in the measurement system and cause measurement errors are previously consumed by the coupled reaction with isocitric acid, metal ions and isocitrate dehydrogenase ( iCDH ); a metal chelating agent is added at the end of elimination to completely terminate the iCDH reaction, simultaneously or thereafter an enzyme or substrate that generates $H_2O_2$ as final reaction product is added, and the amount of $H_2O_2$ thus formed is measured without adverse effect of any other substances.

The enzymes involved in the present invention convert the particular biochemical substance and the endogenous substances, which interfere the result, in order to produce hydrogen peroxide, which is then

used to convert NAD(P)H to NAD(P) to detect the presence of the particular biochemical substance, NAD(P) being regenerated to NAD(P)H if endogenous substances are the converted substances.

The present invention is described below in more details.

The inventive method consists of two reaction systems. The first one is the $H_2O_2$ detection system, which may be expressed by the following formula (1):

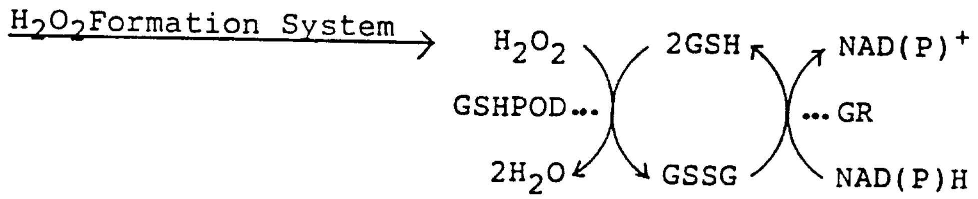


(1)

Hydrogen peroxide is allowed to react with a sufficient amount of GSH in the presence of GSHPOD, GSSG thus formed is reduced by GR, and the resultant decrease in the amount of NAD(P)H is determined by measurement of absorbance at 340 nm. Thus, the present method, in which the decrease in absorbance caused by the consumption of NAD(P)H is measured, assures correct measurement because of no adverse effects of colored substances in test samples and because of the known molar extinction coefficient. In addition, the effect of reducing substances, such as cystein, GSH and ascorbic acid, is minimized because of the sufficient amount of GSH present in the system.

The second reaction system to eliminate the endogenous substances contained in body fluid ( elimination system ) is represented by the following formula (2):

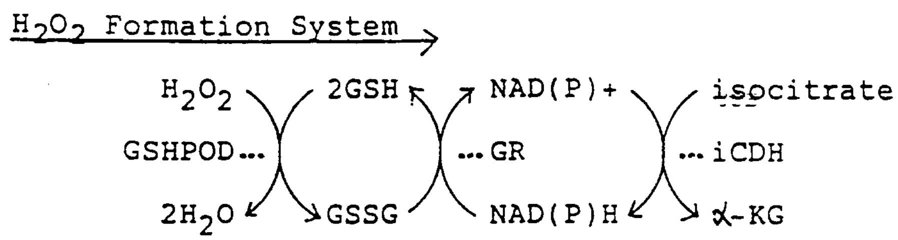


(2)

( wherein α-KG: α-ketoglutaric acid )

Test samples (such as serum and urine) generally contain substances which participate in the reaction system to analyze the biochemical substance being measured ( such as enzyme and substrate ), thus producing measurement errors. These include free glycerol and glycerol 3-phosphate for triglyceride; creatine and sarcosine for creatinine; sarcosine for creatine; free cholesterol for cholesterol esters; choline for phospholipids, such as lecithin, lysolecithin and sphingomyelin; acetyl-CoA for free fatty acids; choline and o-toluoylcholine for choline esterase; maltose and glucose for amylase; allylamine for monoamine oxidase; hypoxanthine for inorganic phosphorus; oxaloacetic and pyruvic acids for transaminase; N-acetylneuraminic and pyruvic acids for sialic acid; xanthine for guanase; and $\beta$-glucose for $\alpha$-glucose. Hydrogen peroxide derived from these endogenous substances is eliminated by GSHPOD and GR, NAD(P)-H thus consumed is regenerated by the coupled reaction of isocitric acid, iCDH and metal ions ( e.g., magnesium and manganese ions ), iCDH is inactivated by a chelating agent, thereby completely terminating the reaction from $NAD(P)^+$ into NAD(P)H, simultaneously or thereafter an enzyme or substrate that generates $H_2O_2$ as final reaction product is then added, and the amount of $H_2O_2$ thus formed is accurately measured without adverse effect of any other substances, the present invention's characteristic existing in this advantage. Furthermore, superoxide dismutase may also be added to the measurement system of this invention.

The metal ions herein include ions of magnesium, manganese, iron, copper, zinc, tin, and calcium.

The chelating agent includes EDTA and salts thereof, 1,2-bis(o-aminophenoxy)ethane-N,N,N',N'-tetraacetic acid and salts thereof, 1,3-diaminopropan-2-ol-N,N,N',N'-tetraacetic acid and salts thereof, diethylenetriamine-N,N,N',N",N"-pentaacetic acid and salts thereof, ethylenediamine-N,N'-diacetic acid and salts thereof, ethylenediamine-N,N'-dipropionic acid and salts thereof, N-hydroxyethylethylenediamine-N,N',N'-triacetic acid and salts thereof, ethylenediamine-N,N,N',N'-tetrakis(methylenephosphonic acid) and salts thereof, glycoletherdiamine-N,N,N',N'-tetraacetic acid and salts thereof, hydroxyethyliminodiacetic acid

and salts thereof, iminodiacetic acid and salts thereof, 1,2-diaminopropane-N,N,N',N'-tetraacetic acid and salts thereof, nitrilotriacetic acid and salts thereof, nitrilotripropionic acid and salts thereof, nitrilotris-(methylenephosphonic acid) and salts thereof, and triethylenetetramine-N,N,N',N'',N''',N'''-hexaacetic acid and salts thereof.

Listed below are typical examples of the $H_2O_2$ formation systems used in the method of invention for the quantitative analysis of substrates and enzymes, which are offered by way of illustration and not by way of limitation.

(1)

$$\text{Cholesterol ester} + H_2O \xrightarrow{\text{Cholesterol esterase}} \text{Free cholesterol} + \text{Fatty acid}$$

$$\text{Free cholesterol} + O_2 \xrightarrow{\text{Cholesterol oxidase}} \Delta^4\text{-Cholestenone} + H_2O_2$$

(2)

$$\begin{bmatrix}\text{Lecithin, Lysolecithin,}\\ \text{Sphingomyelin}\end{bmatrix} \xrightarrow{\text{Phospholipase}} \text{Choline} \xrightarrow{\text{Choline oxidase}} \text{Betaine} + 2H_2O_2$$

(3)

$$\text{Neutral fat} + H_2O \xrightarrow{\text{Lipoprotein lipase}} \text{Glycerol} + \text{Fatty acid}$$

$$\text{Glycerol} + O_2 \xrightarrow{\text{Glycerol oxidase}} \text{Glyceraldehyde} + H_2O_2$$

(4)

$$\text{Neutral fat} + H_2O \xrightarrow{\text{Lipoprotein lipase}} \text{Glycerol} + \text{Fatty acid}$$

$$\text{Glycerol} + \text{ATP} \xrightarrow{\text{Glycerol kinase}} \text{Glycerol-3-phosphate} + \text{ADP}$$

$$\text{Glycerol-3-phosphate} \xrightarrow{\text{Glycerol-3-phosphate oxidase}} \text{Dihydroxyacetone phosphate} + H_2O_2$$

(5)

$$\text{RCOOH} + \text{ATP} + \text{CoA} \xrightarrow{\text{Acyl-CoA synthetase}} \text{Acyl-CoA} + \text{AMP} + \text{PPi}$$

$$\text{Acyl-CoA} + O_2 \xrightarrow{\text{Acyl-CoA oxidase}} \text{2,3-Trans-enoyl-CoA} + H_2O_2$$

(6)

$$\text{Uric acid} + O_2 + H_2O \xrightarrow{\text{Uricase}} \text{Allantoin} + CO_2 + H_2O_2$$

(7)

$$\text{D-Glucose} + O_2 \xrightarrow{\text{Glucose oxidase}} \text{D-Gluconic acid} + H_2O_2$$

(8)

$$\text{o-Toluoylcholine} + H_2O \xrightarrow{\text{Choline esterase}} \text{o-Toluylic acid} + \text{Choline}$$

$$\text{Choline} + O_2 \xrightarrow{\text{Choline oxidase}} \text{Betaine} + 2H_2O_2$$

(9)

$$\text{Maltopentaose} \xrightarrow{\text{Amylase}} \text{Maltotriose} + \text{Maltose}$$

$$\text{Maltose} + \text{Pi} \xrightarrow{\text{Maltose phosphorylase}} \text{Glucose} + \beta\text{-Glucose-1-phosphate}$$

$$\text{Glucose} + O_2 \xrightarrow{\text{Glucose oxidase}} \text{Gluconic acid} + H_2O_2$$

(10)

$$CH_2{=}CHCH_2NH_2 + O_2 + H_2O \xrightarrow{\text{Monoamine oxidase}} CH_2{=}CHCHO + NH_3 + H_2O_2$$

(11)

$$H_3PO_4 + \text{Inosine} \xrightarrow{\text{Purine nucleoside phosphorylase}} \text{Hypoxanthine} + \text{Ribose-1-phosphate}$$

$$\text{Hypoxanthine} + 2O_2 + 2H_2O \xrightarrow{\text{Xanthine oxidase}} \text{Uric acid} + 2H_2O_2$$

(12)

$$\text{Pyruvic acid} + O_2 + Pi \xrightarrow{\text{Pyruvate oxidase}} \text{Acetylphosphoric acid} + CO_2 + H_2O_2$$

(13)

$$\text{L-Lactic acid} + O_2 \xrightarrow{\text{Lactate oxidase}} \text{Pyruvic acid} + H_2O_2$$

(14)

$$\text{L-Aspartic acid} + \alpha\text{-Ketoglutaric acid} \xrightarrow{\text{Glutamic-oxaloacetic transaminase}} \text{Oxaloacetic acid} + \text{L-Glutamic acid}$$

$$\text{Oxaloacetic acid} \xrightarrow{\text{Oxaloacetate decarboxylase}} \text{Pyruvic acid} + CO_2$$

$$\text{Pyruvic acid} + O_2 + Pi \xrightarrow{\text{Pyruvate oxidase}} \text{Acetylphosphoric acid} + CO_2 + H_2O_2$$

(15)

$$\text{L-Alanine} + \alpha\text{-Ketoglutaric acid} \xrightarrow{\text{Glutamic-pyruvic transaminase}} \text{Pyruvic acid} + \text{L-Glutamic acid}$$

$$\text{Pyruvic acid} + O_2 + Pi \xrightarrow{\text{Pyruvate oxidase}} \text{Acetylphosphoric acid} + CO_2 + H_2O_2$$

(16)

$$\alpha\text{-Glucose} \xrightarrow{\text{Mutarotase}} \beta\text{-Glucose}$$

$$\beta\text{-Glucose} + O_2 \xrightarrow{\text{Glucose oxidase}} \text{Gluconic acid} + H_2O_2$$

(17)

$$\text{Creatinine} + H_2O \xrightarrow{\text{Creatinine amidohydrolase}} \text{Creatine}$$

$$\text{Creatine} + H_2O + O_2 \xrightarrow{\text{Creatine amidinohydrolase}} \text{Sarcosine} + \text{Urea}$$

$$\text{Sarcosine} + H_2O + O_2 \xrightarrow{\text{Sarcosine oxidase}} \text{Formaldehyde} + \text{Glycine} + H_2O_2$$

(18)

$$\text{Sialic acid} \xrightarrow{\text{Neuraminidase}} \text{N-Acetylneuraminic acid}$$

$$\text{N-Acetylneuraminic acid} + O_2 \xrightarrow{\text{N-Acetylneuraminate oxidase}} \text{N-Acetyl-D-mannosamine} + \text{Pyruvic acid}$$

$$\text{Pyruvic acid} + O_2 + \text{Pi} \xrightarrow{\text{Pyruvate oxidase}} \text{Acetylphosphoric acid} + CO_2 + H_2O_2$$

(19)

$$\text{Guanine} \xrightarrow{\text{Guanase}} \text{Xanthine} + NH_3$$

$$\text{Xanthine} + O_2 \xrightarrow{\text{Xanthine oxidase}} \text{Uric acid} + H_2O_2$$

The following examples will further illustrate the invention.

Example 1

To 1.5 ml of 0.1M K-$PO_4$ solution (pH 7.5) containing 0.6mM oxonic acid, 2mM GSH, 0.5mM NADPH, 2mM $NaN_3$, 0.5 u/ml GR, 1 u/ml GSHPOD, 1 u/ml iCDH, 5mM isocitric acid and 0.2mM $MgCl_2$, was added 75 µl each of serial dilutions of 120 mg/dl uric acid solution ( test samples ), and the mixture was allowed to stand at 37°C for three to five minutes. 0.1M K-$PO_4$ solution ( pH 7.5 ) containing 9 u/ml uricase and 20mM EDTA-4Na was then added ( 1.5 ml each ), the reaction was allowed to proceed at 37°C, and the decrease in absorbance at 340 nm was measured over a period of three minutes. The change in absorbance per minute ( $\Delta A_{340nm}$/min ) was calculated from the data in the part of high linearity. The result is summarized in the table below.

| Sample No. | 1 | 2 | 3 | 4 | 5 | 6 |
|---|---|---|---|---|---|---|
| Uric acid (mg/dl) | 0 | 24 | 48 | 72 | 96 | 120 |
| Measurements ($\Delta A_{340nm}$/min) | 0.0020 | 0.0415 | 0.0760 | 0.1190 | 0.1567 | 0.1822 |

As can be seen from the table, a linear relationship was obtained at uric acid concentrations up to 120 mg/dl at a sampe:reagent ratio of 1:40.

Example 2

To 1.5 ml of 0.1M potassium phosphate buffer solution ( pH 7.0 ) containing 2mM GSH, 0.5mM NADPH, 2mM $NaN_3$, 0.5 u/ml GR, 2 u/ml iCDH, 10mM isocitric acid, 1 u/ml GSHPOD and 0.2mM $MgCl_2$, was added 20 µl each of serial dilutions ( 0 to 300 mg/dl ) of aqueous cholesterol solution, and the mixture was allowed to stand at 37°C for five minutes. 0.1M potassium phosphate buffer solution ( pH 7.0 ) containing 20mM EDTA-4Na and 6 u/ml cholesterol oxidase was then added ( 1.5 ml each ), the mixture was allowed to stand at 37°Cfor 15 minutes and then at room temperature for about ten minutes, and the absorbance at 340 nm was measured. The result obtained is summarized in the table below.

| Sample No. | 1 | 2 | 3 | 4 | 5 | 6 |
|---|---|---|---|---|---|---|
| Cholesterol (mg/dl) | 0 | 60 | 120 | 180 | 240 | 300 |
| Measurements ($\Delta A_{340nm}$)<br>(mg/dl) | 0.002<br>0 | 0.062<br>57 | 0.130<br>121 | 0.195<br>182 | 0.249<br>233 | 0.312<br>292 |

As can be seen from the table, the amounts of cholesterol in test samples could be measured with high linearity at a sample:reagent ratio of 1:150.

The following equation was used for the calculation of cholesterol concentration:

$$mg/dl = \frac{\Delta A \times 3.02}{6.2 \times 0.02} \times \frac{386.64}{1000} \times 100$$

wherein $\Delta A = A - A_0$ ( $A_0$ is reagent blanks, for which Sample No. 1 is allotted )

6.2 = Absorbance of 1mM NADPH

3.02 = Total volume of reaction system (ml)

0.02 = Volume of test sample (ml)

386.64 = Molecular weight of cholesterol

Example 3

To 2 ml of a reaction mixture obtained from 5 u/ml maltose phosphorylase, 10 u/ml glucose oxidase, 0.2mM $MgCl_2$, 1.5mM GSH, 0.38mM NADPH, 0.75 u/ml GR, 1.5 u/ml GSHPOD, 1.5 u/ml iCDH, 7.5 mM isocitric acid and 0.1 M phosphate buffer solution ( pH 7.2 ), was added 50 µl each of patients' serum samples of known amylase activity ( 152 u/l for Sample A, 329 u/l for Sample B, 192 u/l for Sample C, 211 u/l for Sample D and 436 u/l for Sample E ), and the mixture was allowed to stand at 37°C for three to five minutes. 0.1 M phosphate buffer solution ( pH 7.2 ) containing 30 mM maltopentaose and 30mM EDTA was then added ( 1 ml each ), the reaction was allowed to proceed at 37°C, and the change in absorbance at 340 nm per minute was measured. The result obtained in summarized in the table below.

| Sample No. | A | B | C | D | E |
|---|---|---|---|---|---|
| Amylase activity (u/l) | 152 | 329 | 192 | 211 | 436 |
| Measurements ($\Delta A_{340nm}$/min)<br>Activity (u/l) | 0.015<br>148 | 0.035<br>344 | 0.020<br>197 | 0.021<br>207 | 0.045<br>443 |

The following equation was used for the calculation of amylase activity:

$$u/l = \frac{\Delta A_{340nm}/min \times 3.05}{6.2 \times 0.05} \times 1000$$

wherein
3.05: Final volume of reaction system (ml)
0.05: Volume of test sample (ml)
6.2: Molar extinction coefficient of NADPH (mM)

Example 4

To 1 ml of 0.1M phosphate buffer solution ( pH 7.8 ) containing 20 u/ml creatine amidinohydrolase, 0.5 u/ml sarcosine oxidase, 2mM GSH, 0.4mM NADPH, 0.5 u/ml GR, 1 u/ml GSHPOD, 2 u/ml iCDH, 5 mM isocitric acid and 0.2mM $MgCl_2$, was added 20 µl each of serial dilutions of aqueous creatinine solution ( 0 to 100 mg/dl ), and the mixture was allowed to stand at 37°C for five minutes. 0.1M phosphate buffer solution ( pH 7.8 ) containing 20mM EDTA-4Na and 32 u/ml creatinine amidohydrolase was then added ( 1 ml each ), the reaction was allowed to proceed at 37°C, the change in absorbance at 340 nm was measured from two minutes after the start of reaction over a period of 30 seconds, and the change per minute was calculated. The result obtained is summarized in the table below.

| Dilution (mg/dl) | 0 | 20 | 40 | 60 | 80 | 100 |
|---|---|---|---|---|---|---|
| Measurements: $\Delta A_{340nm}$/min (mAbs) | 0.1 | 39.2 | 72.6 | 111.3 | 124.9 | 169.9 |

As can be seen from the table, a good linear relationship was observed.

Example 5

Test samples of concentrations as shown in the table below were prepared using a standard triolein solution ( 250 mg/dl ) and a glycerol solution.

| Sample No. | 1 | 2 | 3 | 4 | 5 | 6 | 7 | 8 | 9 | 10 | 11 | 12 |
|---|---|---|---|---|---|---|---|---|---|---|---|---|
| Triolein (mg/dl) | 0 | 50 | 100 | 150 | 200 | 250 | 0 | 50 | 100 | 150 | 200 | 250 |
| Glycerol (mg/dl) | 0 | 0 | 0 | 0 | 0 | 0 | 5 | 5 | 5 | 5 | 5 | 5 |

To 1.5 ml of 50mM Tris-HCl buffer solution ( pH 7.8 ) containing 0.36 u/ml glycerol kinase, 2mM GSH, 0.2mM $MgCl_2$, 1 u/ml iCDH, 5mM isocitric acid, 5 u/ml glycerol-3-phosphate oxidase, 0.5mM NADPH, 0.5 u/ml GR, 1 u/ml GSHPOD and 0.002mM ATP, was added 20 µl each of the test samples prepared above, and the mixture was allowed to stand at 37°C for ten minutes. 50mM Tris-HCl buffer solution ( pH 7.8 ) containing 10 u/ml lipoprotein lipase and 20mM EDTA-4Na was then added ( 1.5 ml each ), the mixture was allowed to stand at 37°C for 15 minutes and then at room temperature for about ten minutes, and the absorbance at 340 nm was measured. The result obtained is summarized in the table below.

| Sample No. | 1 | 2 | 3 | 4 | 5 | 6 |
|---|---|---|---|---|---|---|
| Measurements ($\Delta A_{340nm}$) | 0.002 | 0.022 | 0.047 | 0.074 | 0.093 | 0.118 |
| Triolein (mg/dl) | 0.0 | 43.1 | 107.8 | 155.2 | 196.2 | 250.2 |

| Sample No. | 7 | 8 | 9 | 10 | 11 | 12 |
|---|---|---|---|---|---|---|
| Measurements ($\Delta A_{340nm}$) | 0.002 | 0.025 | 0.050 | 0.070 | 0.091 | 0.116 |
| Triolein (mg/dl) | 0.0 | 49.6 | 103.5 | 146.6 | 191.9 | 245.8 |

The amount of glycerol was calculated from the value of $\Delta A_{340nm}$ from which the reagent blanks had been subtracted, assuming the molecular weight of triolein to be 885.45.

It was thus demonstrated that the amount of triolein can be measured with no effect of glycerol added.

Effects Achieved by the Invention

The method of this invention comprises a new step of eliminating endogenous substances in the NAD(P)H regeneration system using iCDH, which is followed by measurement of the amount of hydrogen peroxide formed, and hence has outstanding advantages over the conventional methods as described below. This invention provides a new method of analyzing biochemical substances using hydrogen peroxide in the detection system, wherein enzymes or enzyme groups, or substrates or substrate groups, required for the reaction to generate hydrogen peroxide are admixed to a test sample, together with glutathione peroxidase, glutathione reductase, isocitric acid, NAD(P)H, metal ions and isocitrate dehydrogenase, thereby consuming endogenous substances contained in body fluid that participate in the reaction system to measure the biochemical substances (substrates, enzymes, etc.) being analyzed and hence produce measurement errors; a metal chelating agent is then added to terminate the reaction of isocitrate dehydrogenase; simultaneously or thereafter an enzyme or substrate that generates hydrogen peroxide as reaction product is added, followed by measurement of the amount of hydrogen peroxide thus formed. The factors interfering the measurment can thus be removed, ensuring correct measurement. Since living bodies contain a variety of such interfering factors, the method of this invention is particularly useful for the quantitative analysis of biochemical substances in samples taken from organisms, and can be advantageously applied to various types of diagnosis.

This method also provides a new type of reagent whereby oxidase is allowed to act upon substances contained in a test sample or substances formed by specific reactions, thus converting all the enzymes or substrates participating in the reaction system into hydrogen peroxide.

Such outstanding features of the method of this invention are based on the facts that decrease in the amount of NAD(P)H with definite molar extinction coefficient is used for measurement, that the measurement is little affected by the color of test samples, pH of the reaction system and other factors, and that the coupled reactions of iCDH and GR are utilized to allow regeneration of reduced-form glutathione and NAD(P)H, thus avoiding background fluctuations caused by consumption of the endogenous interfering substances. The method of this invention is also applicable to automatic analyzers through the end-point and rate assay, opening the way to rapid, simple and correct measurement.

## Claims

**1.** Enzyme assay for the quantitative analysis of a biochemical substance which, when subjected to a predetermined enzyme or substrate, undergoes a reaction which, either directly or indirectly after adding one or more secondary enzymes or substrates, yields hydrogen peroxide as a reaction product, comprising:

adding to a test sample glutathione (GSH), glutathione peroxidase (GSHPOD), glutathione reductase (GR), NADPH or NADH, isocitric acid, metal ions, isocitrate dehydrogenase, and said one or more secondary enzymes or substrates, thereby consuming any endogenous substances which may generate hydrogen peroxide upon reaction with any of said added substances, or that would otherwise eventuate in the reduction of NADPH or NADH to $NADP^+$ or $NAD^+$, and regenerating NADPH or NADH at the same time;

inactivating the isocitrate dehydrogenase;

adding, simultaneously with or after said inactivating step, said predetermined enzyme or substrate that generates hydrogen peroxide as reaction product when reacting with the biological substance directly or indirectly after further reaction with said one or more secondary substances or enzymes, whereby any formed hydrogen peroxide will be subjected to the action of the GSHPOD in the presence of the GSH to produce oxidized glutathione (GSSG) and any formed GSSG will be subjected to the action of the GR to produce $NAD^+$ or $NAD^+$ from the NADPH or NADH previously added or

regenerated; and

measuring at 340 nm any decrease of the NADPH or NADH.

2. Assay in accordance with claim 1, wherein said inactivating step comprises adding a sufficient amount of a chelating agent.

**Patentansprüche**

1. Enzymuntersuchung für die quantitative Analyse einer biochemischen Substanz, die, wenn sie einem vorher festgelegten Enzym oder Substrat unterworfen wird, eine Reaktion eingeht, welche entweder direkt oder indirekt nach Zugabe von einem oder mehreren sekundären Enzymen oder Substraten Wasserstoffperoxid als Reaktionsprodukt ergibt, dadurch gekennzeichnet, daß man

zu einer Testprobe Glutathion (GSH), Glutathionperoxidase (GSHPOD),Glutathionreduktase (GR), NADPH oder NADH, Isocitronensäure, Metallionen, Isocitratdehydrogenase und wie genannt eines oder mehrere sekundäre Enzyme oder Substrate hinzugibt, wodurch die betreffenden endogenen Substanzen verbraucht werden, welche bei Reaktion mit irgendeiner der zugesetzten Substanzen Wasserstoffperoxid erzeugen können, oder die ansonsten zur Reduktion von NADPH oder NADH zu $NADP^+$ oder $NAD^+$ führen würden, und gleichzeitig NADPH oder NADH regeneriert werden;

die Isocitratdehydrogenase inaktiviert;

gleichzeitig mit der Inaktivierungsstufe oder danach das vorher festgelegte Enzym oder Substrat hinzugibt, welches Wasserstoffperoxid als Reaktionsprodukt erzeugt, wenn es mit der biologischen Substanz umgesetzt wird direkt oder indirekt nach weiterer Reaktion mit wie genannt dem einen oder den mehreren sekundären Substanzen oder Enzymen,

wobei das gebildete Wasserstoffperoxid der Einwirkung von GSHPOD in Gegenwart von GSH unterworfen wird unter Erzeugung von oxidiertem Glutathion (GSSG), und das gebildete GSSG der Einwirkung von GR unterworfen wird zur Erzeugung von $NADP^+$ oder $NAD^+$ aus NADPH oder NADH, wie zuvor hinzugegeben oder regeneriert; und

die Verringerung von NADPH oder NADH bei 340 nm mißt.

2. Enzymuntersuchung nach Anspruch 1, wonach die Inaktivierungsstufe die Zugabe einer hinreichenden Menge eines Chelatisierungsmittels einschließt.

**Revendications**

1. Dosage enzymatique pour l'analyse quantitative d'une substance biochimique qui, lorsqu'elle est soumise à un enzyme ou substrat pré-déterminé, subit une réaction qui, soit directement, soit indirectement après addition d'un ou plusieurs enzymes ou substrats secondaires, fournit du péroxyde d'hydrogène en tant que produit de réaction, dans lequel :

on ajoute à un échantillon d'essai du glutathion (GSH), de la péroxydase de glutathion (GSHPOD), de la réductase de glutathion (GR), du NADPH ou du NADH, de l'acide isocitrique, des ions métalliques, de la déshydrogénase d'isocitrate et un ou plusieurs enzymes ou substrats secondaires, de façon à consumer des substances endogènes quelconques qui peuvent générer du péroxyde d'hydrogène lors de la réaction avec une quelconque desdites substances ajoutées, ou qui pourraient intervenir par ailleurs dans la réduction du NADPH ou NADH en $NADP^+$ ou $NAD^+$, et on regénère NADPH ou NADH en même temps ;

on inactive la déshydrogénase d'isocitrate ;

on ajoute simultanément avec ou après ladite étape d'inactivation, ledit enzyme ou substrat prédéterminé qui produit du péroxyde d'hydrogène en tant que produit de réaction, lorsqu'il réagit avec la substances biologique, directement ou indirectement après une autre réaction avec une ou plusieurs substances ou enzymes secondaires, de façon que le péroxyde d'hydrogène formé soit soumis à l'action du GSHPOD en présence du GSH afin d'obtenir un glutathion oxydé (GSSG) et ce dernier produit formé sera soumis à l'action du GR afin d'obtenir $NADP^+$ ou $NAD^+$ à partir du NADPH ou NADH ajouté auparavant ou régénéré ; et

on mesure à 340 nm une diminution quelconque du NADPH ou NADH.

2. Dosage selon la revendication 1, dans lequel ladite étape d'inactivation consiste à ajouter une quantité suffisante d'un agent chélatant.